(19) Europäisches Patentamt / European Patent Office / Office européen des brevets

(11) **EP 3 517 034 A1**

(12) **EUROPEAN PATENT APPLICATION**

(43) Date of publication:
**31.07.2019 Bulletin 2019/31**

(51) Int Cl.:
**A61B 5/1455** *(2006.01)*          **A61B 5/00** *(2006.01)*
**A61B 5/024** *(2006.01)*

(21) Application number: **18153213.6**

(22) Date of filing: **24.01.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **DE HAAN, Gerard
  5656 AE Eindhoven (NL)**
• **SERTEYN, Aline Anne Marie
  5656 AE Eindhoven (NL)**
• **VAN GASTEL, Mark J.H.
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE, SYSTEM AND METHOD FOR DETERMINING AT LEAST ONE VITAL SIGN OF A SUBJECT**

(57)    The present invention relates to a device, system and method for determining at least one vital sign of a subject. The device (130) comprises an input interface (131) configured to obtain at least three detection signals derived from detected electromagnetic radiation transmitted through or reflected from a skin region of a subject, wherein each detection signal comprises wavelength-dependent reflection or transmission information in a different wavelength channel, a transformation unit (132) configured to transform the at least three detection signals into at least three transformed detection signals by temporally normalizing the at least three detection signals or applying a non-linear transfer function on the at least three detection signals, a projection unit (133) configured to project the at least three transformed detection signals into at least two projected signals in a subspace orthogonal to a predetermined distortion direction, and a vital sign determination unit (134) configured to determine a vital sign from said at least two projected signals by indirectly determining the relative strength of a pulsatile component in said at least two projected signals.

130

FIG.3

EP 3 517 034 A1

**Description**

FIELD OF THE INVENTION

The present invention relates to a device, system and method for determining at least one vital sign of a subject.

BACKGROUND OF THE INVENTION

Vital signs of a person, for example the heart rate (HR), the respiration rate (RR) or the (peripheral or pulsatile) blood oxygen saturation (SpO2; it provides an estimate of the arterial blood oxygen saturation SaO2), serve as indicators of the current state of a person and as powerful predictors of serious medical events. For this reason, vital signs are extensively monitored in inpatient and outpatient care settings, at home or in further health, leisure and fitness settings.
One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.
Photoplethysmography (PPG) is an optical measurement technique that evaluates a time-variant change of light reflectance or transmission of an area or volume of interest. PPG is based on the principle that the blood absorbs light more than the surrounding tissue, so variations in blood volume with every heartbeat affect the transmission or reflectance correspondingly. Besides information about the pulse rate (heart rate), a PPG waveform (also called PPG signal) can comprise information attributable to further physiological phenomena such as the respiration. By evaluating the transmittance and/or reflectance at different wavelengths (typically red and infrared), the blood oxygen saturation can be determined.
Conventional pulse oximeters (also called contact PPG device herein) for measuring the pulse rate and the (arterial) blood oxygen saturation of a subject are attached to the skin of the subject, for instance to a fingertip, earlobe or forehead. Therefore, they are referred to as 'contact' PPG devices. Although contact PPG is basically regarded as a non-invasive technique, contact PPG measurement is often experienced as being unpleasant and obtrusive, since the pulse oximeter is directly attached to the subject and the cables limit the freedom to move and might hinder a workflow.
Non-contact, remote PPG (rPPG) devices (also called camera-based device) for unobtrusive measurements have been proposed in the last decade. Remote PPG utilizes light sources or, in general, radiation sources, disposed at a distance from the subject of interest. Similarly, a detector, e.g. a camera or a photodetector, can be disposed at a distance from the subject of interest. Therefore, remote photoplethysmographic systems and devices are considered unobtrusive and well suited for medical as well as non-medical everyday applications.
Using the PPG technology, vital signs can be measured. Vital signs are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or strict accuracy requirements, where an improved robustness and accuracy of the vital sign measurement devices and methods is required, particularly for the more critical healthcare applications.
The measurement of one of the important vital signs, the blood oxygen saturation (SpO2), has recently shown to be feasible with camera-based rPPG. SpO2 measurements require an accurate detection of relative pulsatilities, i.e. the normalized amplitude of the pulsatile signal (AC/DC) in different wavelength channels. There are two major threats to the accuracy in remote measurement. The first is that the pulsatilities are low compared to the noise, while the second is that ballistocardiographic (BCG) motion modifies the relative pulsatilities in each channel.
Next to oxygen saturation, the saturation of other arterial blood gasses and blood species, e.g. HBCO, MetHB, HBCO2, bilirubin, may be obtained using the same (somewhat adapted) technique, and suffer from the same threat that the current invention aims to solve. The arterial blood components are selected with this technique because only the arterial blood is assumed to pulsate at the rhythm of the cardiac activity. It is hypothesized that, similarly, the respiratory cycle induces volume variations in the venous system, implying that the relative strength of the pulsations at the respiratory rhythm in different wavelengths may be used to analyze the venous blood components, e.g. to estimate the venous oxygen saturation (SvO2). In this similar case, the threats are not the ballistocardiographic motions, but rather the respiratory motion of the skin-site where the measurements are done.
In conclusion, there is a need for an improved device, system and method for determining at least one vital sign of a subject to obtain results with higher reliability even in case of noise and/or motion.

SUMMARY OF THE INVENTION

It is an object of the present invention to provide a device, system and method for determining at least one vital sign of a subject by which results with higher reliability, even in case of noise and/or motion, can be achieved.

In a first aspect of the present invention, a device for determining at least one vital sign of a subject is presented comprising:

- an input interface configured to obtain at least three detection signals derived from detected electromagnetic radiation transmitted through or reflected from a skin region of a subject, wherein each detection signal comprises wavelength-dependent reflection or transmission information in a different wavelength channel,
- a transformation unit configured to transform the at least three detection signals into at least three transformed detection signals by temporally normalizing the at least three detection signals or applying a non-linear transfer function on the at least three detection signals,
- a projection unit configured to project the at least three transformed detection signals into at least two projected signals in a subspace orthogonal to a predetermined distortion direction, and
- a vital sign determination unit configured to determine a vital sign from said at least two projected signals by indirectly determining the relative strength of a pulsatile component in said at least two projected signals.

In a further aspect of the present invention a system for determining at least one vital sign of a subject is presented comprising:

- a detector for detecting electromagnetic radiation transmitted through or reflected from a skin region of a subject and for deriving at least three detection signals from the detected electromagnetic, wherein each detection signal comprises wavelength-dependent reflection or transmission information in a different wavelength channel, and
- a device as disclosed herein for determining at least one vital sign of the subject from the at least three detection signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

One main problem addressed by the present invention is that BCG motion modifies the relative pulsatilities in each wavelength channel. More particularly, as the heart contracts, it pushes a volume of blood, through the aorta and the carotid arteries, into the head. This periodic excitation of the mass-spring system that models the head (mass) and the muscles (spring) that keep it upright, leads to a periodic motion of the head. BCG motions, however, may occur at all body parts, and may also be caused by superficial arteries that cause the skin to move in the rhythm of the pulse.

As the periodic motion, relative to a light source, causes a periodic brightness variation registered by the detector of the radiation, e.g. a vital signs camera, it modifies the relative pulsatilities in all wavelength channels. This modification can lead to an enhanced pulsatility, but also to a decreased pulsatility, depending on the phase of the BCG-motion-induced reflection variations relative to the phase of the PPG signal, i.e. the pulsatile component derived from the detection signals. This phase difference may even change over time, as the muscle tension is not fixed, but also varies. Consequently, the SpO2 value, derived from the relative pulsatilities in the wavelength channels, and also the value of other blood species/components (if measured as the intended vital sign), are affected and can be increased or decreased depending on the momentary phase difference between BCG-motion-induced reflection variations and PPG-induced skin-absorption variations. A similar problem occurs for respiratory motions of the skin if the vital sign is measured at the respiratory frequency (e.g. SvO2).

In this context, it shall be noted that the term "vital sign" shall be understood broadly in the sense of "physiological parameter" including not only vital signs in the strict sense, but also other physiological parameters like SvO2 or SaO2. Said vital sign determination unit may thus be configured to determine as vital sign an indicator or concentration of blood components or species, in particular SpO2, SvO2, HBO2, HBCO, METHB, Bilirubin, and wherein the pulsatile component reflects the cardiac cycle or the respiratory cycle.

The present invention makes use of the recognition that motion, in uniform illumination conditions, affects the relative pulsatilities in the different wavelength channels (substantially) identically, i.e. it only changes the intensity of the skin-reflected light but not the color. This means that the effect of BCG (or other relevant motion) is absent in signals that result from linearly weighted combinations of the wavelength channels when the weights add up to zero (signals in the space orthogonal to the skin color). In US 2015/0366492 A1 a device and a method are described according to which two pairs of wavelength-difference signals are used. These difference signals have the characteristic that their sum is zero, however, with a different weighted combination as proposed according to the present invention, an even further improvement can be achieved. Furthermore, using a ratio-of-ratios method to establish the SpO2 as proposed in WO 2015/197383 A1 is vulnerable to noise. Since the signals in the space orthogonal to the skin color are even more noisy (the (correlated) pulsatility decreases due to the differencing, while the (uncorrelated) noises add up) than they already are in the original wavelength channels, an indirect measurement is used according to the present invention to obtain acceptable results.

With respect to the predetermined direction, it is noted that the direction of choice is the direction [1, ..., 1], i.e. the direction where all detection signal values are equal. Motion typically causes the same (relative) variation in all wavelength channels, especially if the ambient illumination is uniform.

According to an embodiment, said projection unit is configured to project the at least three transformed detection signals into the at least two projected signals to optimize the sensitivity of the vital sign and the strength of the pulsatile component. This further improves elimination of the BCG motion, which is provided by the projection.

The projection unit may particularly be configured to project the at least three transformed detection signals into the at least two projected signals by optimizing the selection of projection axes using as optimization criteria that the relative pulsatilities of the at least two projected signals are affected at all and that are affected significantly differently by variations of the vital sign. Particularly, in the case of SpO2, it is preferred that the relative pulsatilities of the two projected signals are affected significantly differently by SpO2 variations, otherwise, the ratio of the pulsatile strength of the two projected signals does not provide a correct measure of SpO2. Additionally, there are projections possible that almost have no pulsatility at all, which is also unfavorable. These criteria together can be used to optimize the selection of the projection axes.

According to another embodiment, said transformation unit is configured to divide time samples of the individual detection signals by a division value derived from said time samples to determine the at least three transformed detection signals. This provides for a temporal normalization.

In another embodiment said transformation unit is configured to transform time samples of the detection signals using a logarithm function to determine the at least three transformed detection signals.

The wavelength channels may be in a wavelength interval between 400nm and 1000nm, in particular between 630nm and 940nm. In this wavelength interval, the vital sign can be determined reliably.

In a preferred embodiment, said input interface is configured to obtain three detection signals in wavelength channels centered on i) 660nm or 760nm, ii) 800nm, and iii) 840nm or 900nm.

Further, said projection unit may be configured to project the at least three transformed detection signals into at least two projected signals by a weighted summation of the transformed detection, wherein the sum of the weights equals zero. If this holds for all (at least two) projected signals, these lines span a subspace that is orthogonal to said predetermined distortion direction.

A good result is obtained in an embodiment in which said projection unit is configured to project three transformed detection signals Ct1, Ct2, Ct3 into two projected signals $P_1$, $P_2$ as:

$$P_1 = [-1.0465 \ 0.9459 \ 0.1006]*[Ct1 \ Ct2 \ Ct3];$$

$$P_2 = [-0.8452 \ -1.1472 \ 1.9924]* [Ct1 \ Ct2 \ Ct3].$$

Said vital sign determination unit may be configured to indirectly determine the relative strength of a pulsatile component in said at least two projected signals by use of a blood-volume pulse vector (PBV) based method or adaptive PBV (APBV) method. In particular, the relative strength of a pulsatile component may be determined in said at least two projected signals by determining the signature vector, PBV_best, from a set of candidate signature vectors, PBV_c, that provides the best quality pulsatile signal according to a criterion (adaptive PBV method), where PBV_best identifies the relative pulsatilities from which the vital sign is derived. Hence, preferably the APBV method is used to establish the relative pulsatilities. For instance, two difference signals (a*Sa+b*Sb, with a=-b, and a*Sa+c*Sc, with a=-c) may be used. The proposed indirect measurement is advantageous compared to a direct measurement, which may often fail and provides a lower SNR.

Generally, a PPG signal results from variations of the blood volume in the skin. Hence, the variations give a characteristic pulsatility "signature" when viewed in different spectral components of the reflected/transmitted light. This "signature" is basically resulting as the contrast (difference) of the absorption spectra of the blood and that of the blood-less skin tissue. If the detector, e.g. a camera or sensor, has a discrete number of color channels, each sensing a particular part of the light spectrum, then the relative pulsatilities in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that, if this signature vector is known, then a motion-robust pulse signal extraction on the basis of the color channels and the signature vector is possible. For the quality of the pulse signal, it is essential though that the signature vector is accurate, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, whose details are also herein incorporated by reference.

The characteristic wavelength-dependency of the PPG signal varies when the composition of the blood changes. Particularly, the oxygen saturation of the arterial blood has a strong effect on the light absorption in the wavelength range between 620nm and 780nm. This changing signature for different SpO2 values leads to relative PPG pulsatility that depends on the arterial blood oxygen saturation. This dependency can be used to realize a motion-robust remote SpO2 monitoring system that has been named adaptive PBV method (APBV) and is described in detail in M. van Gastel, S. Stuijk and G. de Haan, "New principle for measuring arterial blood oxygenation, enabling motion-robust remote monitoring", Nature Scientific Reports, Nov. 2016. The description of the details of the APBV method in this document is also herein incorporated by reference.

The proposed device may further comprise an output unit configured to output the vital sign. The output unit may e.g. be a user interface like a display, computer or loudspeaker. Still further, the proposed device may comprise a control unit configured to generate, based on the vital sign, an alarm control signal for controlling an alarm unit configured to issue an alarm and to output the generated alarm control signal.

BRIEF DESCRIPTION OF THE DRAWINGS

These and other aspects of the invention will be apparent from and elucidated with reference to the embodiment(s) described hereinafter. In the following drawings

    Fig. 1 shows a diagram of the relative PPG amplitude in three different wavelengths;
    Fig. 2 shows a schematic diagram of an embodiment of a system according to the present invention;
    Fig. 3 shows a schematic diagram of a first embodiment of a device according to the present invention;
    Fig. 4A shows values of two optimization criteria for rotations on the plane orthogonal to intensity variations;
    Fig. 4B shows the value of the combined, equally weighted optimization features;
    Fig. 4C shows the calibration curve for the optimal projection;
    Fig. 5A shows DC-normalized signals from three wavelength channels;
    Fig. 5B shows the corresponding spectra;
    Fig. 5C shows a spectrogram of the 890nm channel;
    Fig. 5D shows a signal after projection;
    Fig. 5E shows a corresponding spectrum after amplitude tuning; and
    Fig. 5F shows a spectrogram from the full recording.

DETAILED DESCRIPTION OF THE EMBODIMENTS

Recently, significant improvements to the motion robustness of remote PPG monitoring technique have been achieved. The essential insight enabling separation of motion-induced signal variations and the actual PPG variations is that motion affects all wavelength channels (typical PPG monitoring systems use at least two wavelength channels) equally, i.e. the relative signal strength of motion-induced variation is identical in all channels while the PPG-based variations are clearly wavelength dependent. The wavelength-dependency of the PPG signal is mainly caused by the absorption-contrast between blood and skin that varies with the wavelength. In addition, it also depends to a minor extent on the penetration depth, and scattering properties of light in human skin that vary with the wavelength.

The characteristic wavelength-dependency of the PPG signal can be used as a signature, enabling motion-robust PPG measurement. The number of independent distortions that can be suppressed equals the number of wavelength channels minus one. This so-called PBV method is described in the above cited paper of G. de Haan and A. van Leest, "Improved motion robustness of remote-PPG by using the blood volume pulse signature", Physiol. Meas. 35 1913, 2014.

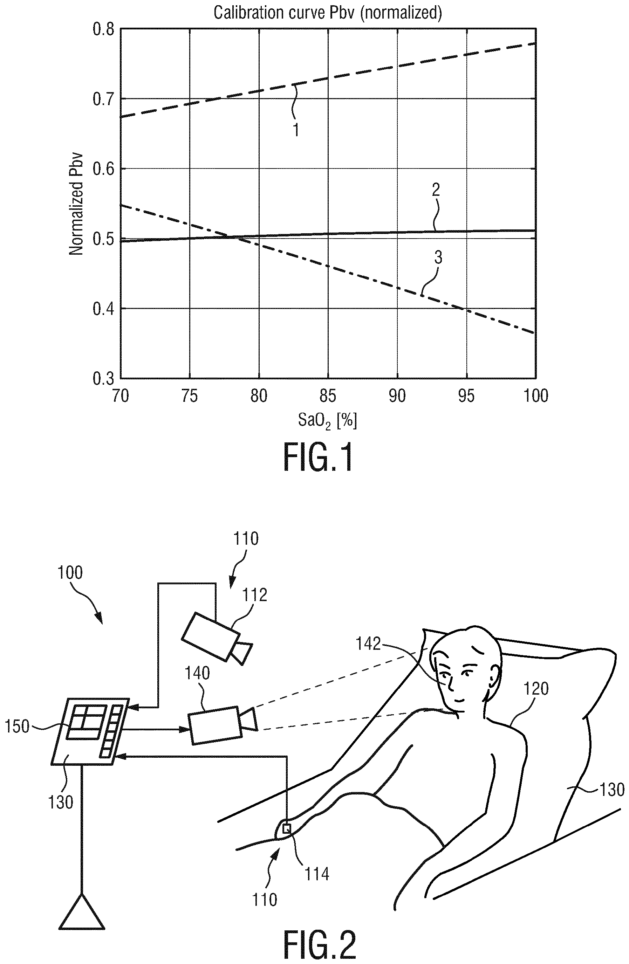

The characteristic wavelength-dependency of the PPG signal varies when the composition of the blood changes. Particularly, the oxygen saturation of the arterial blood has a strong effect on the absorption in the wavelength range between 620nm and 780nm. This changing signature for different SpO2 values leads to relative PPG pulsatility that depends on the arterial blood oxygenation (SaO2) as illustrated in Fig. 1 for three wavelengths in near infrared, in particular for $\lambda_1$=760nm (signal line 1), $\lambda_2$=800nm (signal line 2) and $\lambda_3$=905nm (signal line 3). This dependency can be used to realize a motion-robust remote SpO2 monitoring system that has been named adaptive PBV method (APBV) and is described in the above cited paper of M. van Gastel, S. Stuijk and G. de Haan, "New principle for measuring arterial blood oxygenation, enabling motion-robust remote monitoring", Nature Scientific Reports, Nov. 2016.

It shall be noted here that SaO2 is the actual arterial oxygenation level as would be measured invasively by drawing blood and subsequent analysis. SpO2 is the result from an optical measurement, which is no more than a substitute for the actual measurement, which, however, has been proven to be clinically relevant under certain conditions, be it less accurate, i.e. SpO2 is the estimation result using photoplethysmography.

Fig. 2 shows a schematic diagram of an embodiment of a system 100 according to the present invention. The system 100 comprises a detector 110 for detecting electromagnetic radiation transmitted through or reflected from a skin region of a subject 120 and for deriving at least three detection signals 210 from the detected electromagnetic. Each detection signal 210 comprises wavelength-dependent reflection or transmission information in a different wavelength channel. The system 100 further comprises a device 130 for determining at least one vital sign of the subject from the at least three detection signals 210. The subject 120, in this example a patient, lies in a bed 130, e.g. in a hospital or other healthcare facility, but may also be a neonate or premature infant, e.g. lying in an incubator, or person at home or in a different environment.

There exist different embodiments for a detector for detecting electromagnetic radiation transmitted through or reflected from a subject, which may alternatively (which is preferred) or together be used. In the embodiment of the system 100 two different embodiments of the detector are shown and will be explained below. Both embodiments of the detector are configured for deriving detection signals from the detected electromagnetic radiation, wherein each detection signal comprises wavelength-dependent reflection or transmission information in a different wavelength channel. Hereby, optical filters may be used which are preferably different, but their filter bandwidth can be overlapping. It is sufficient if their wavelength-dependent transmission is different.

In one embodiment the detector comprises a camera 112 (also referred to as imaging unit, or as camera-based or remote PPG sensor) including a suitable photosensor for (remotely and unobtrusively) capturing image frames of the subject 120, in particular for acquiring a sequence of image frames of the subject 120 over time, from which photoplethysmography signals can be derived. The image frames captured by the camera 112 may particularly correspond to a video sequence captured by means of an analog or digital photosensor, e.g. in a (digital) camera. Such a camera 112 usually includes a photosensor, such as a CMOS or CCD sensor, which may also operate in a specific spectral range (visible, IR) or provide information for different spectral ranges. The camera 112 may provide an analog or digital signal. The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color or pseudo-color (in NIR)). The image frames include at least some image pixels being representative of a skin portion of the subject. Thereby, an image pixel may correspond to one photosensitive element of a photo-detector and its (analog or digital) output or may be determined based on a combination (e.g. through binning) of a plurality of the photosensitive elements.

In another embodiment the detector comprises one or more optical photoplethysmography sensor(s) 114 (also referred to as contact PPG sensor(s)) configured for being mounted to a skin portion of the subject 120 for acquiring photoplethysmography signals. The PPG sensor(s) 114 may e.g. be designed in the form of a finger-clip or as a wrist-worn wearable device for measuring the blood oxygen saturation or a heart rate sensor for measuring the heart rate, just to name a few of all the possible embodiments.

When using a camera 112 the system 100 may further optionally comprise a light source 140 (also called illumination source), such as a lamp, for illuminating a region of interest 142, such as the skin of the patient's face (e.g. part of the cheek or forehead), with light, for instance in a predetermined wavelength range or ranges (e.g. in the red, green and/or infrared wavelength range(s)). The light reflected from said region of interest 142 in response to said illumination is detected by the camera 112. In another embodiment no dedicated light source is provided, but ambient light is used for illumination of the subject 120. From the reflected light, only light in a number of desired wavelength ranges (e.g. green and red or infrared light, or light in a sufficiently large wavelength range covering at least two wavelength channels) may be detected and/or evaluated.

The device 130 is further connected to an interface 150 for displaying the determined information and/or for providing medical personnel with an interface to change settings of the device 130, the camera 112, the PPG sensor(s) 114, the light source 140 and/or any other parameter of the system 100. Such an interface 150 may comprise different displays, buttons, touchscreens, keyboards or other human machine interface means.

A system 100 as illustrated in Fig. 2 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 130, the camera 112, the PPG sensor(s) 114 and the interface 150 may work via a wireless or wired communication interface. Other embodiments of the present invention may include a device 130, which is not provided standalone, but integrated into the camera 112 or the interface 150.

Typically, the electromagnetic radiation is in the range of 400nm to 1000nm for pulse, respiration and blood oxygen saturation measurement, particularly in the range of 620nm to 920nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases).The detection signals may be acquired by a photo-sensor (array) in direct contact with the skin and/or using a video camera remotely sensing the subject's skin.

An embodiment uses the PBV method to extract a pulse signal (or a respiratory signal) from two or more different combinations of three, or more, wavelength channels, e.g. from [$\lambda 1$, $\lambda 2$], from [$\lambda 1$, $\lambda 3$], and/or from [$\lambda 1$, $\lambda 2$, $\lambda 3$], where [$\lambda 1$, $\lambda 2$, $\lambda 3$] can be the red-green-blue channels of an RGB-video camera, or different wavelength (intervals) selected in the near infrared region, e.g. [760nm, 800nm, 905nm].

In the following some basic considerations with respect to the PBV method shall be briefly explained, using RGB-wavelength channels as an example.

The beating of the heart causes pressure variations in the arteries as the heart pumps blood against the resistance of the vascular bed. Since the arteries are elastic, their diameter changes in synchrony with the pressure variations. These diameter changes occur even in the smaller vessels of the skin, where the blood volume variations cause a changing absorption of the light.

The unit length normalized blood volume pulse vector (also called signature vector) is defined as PBV, providing the relative PPG-strength in the red, green and blue camera signal, i.e.

$$\vec{P}_{bv} = \frac{\left[\sigma(\vec{R}_n), \sigma(\vec{G}_n), \sigma(\vec{B}_n)\right]}{\sqrt{\sigma^2(\vec{R}_n) + \sigma^2(\vec{G}_n) + \sigma^2(\vec{B}_n)}}$$

with $\sigma$ indicating the standard deviation.

To quantify the expectations, the responses $H_{red}(w)$, $H_{green}(w)$ and $H_{blue}(w)$ of the red, green and blue channel, respectively, were measured as a function of the wavelength w, of a global-shutter color CCD camera, the skin reflectance of a subject, $\rho_s(w)$, and used an absolute PPG-amplitude curve PPG(w). From these curves, shown e.g. in Fig. 2 of the above cited paper of de Haan and van Leest, the blood volume pulse vector PBV is computed as:

$$\vec{P}_{bv}^T = \begin{bmatrix} \dfrac{\int\limits_{w=400}^{700} H_{red}(w)I(w)PPG(w)\,dw}{\int\limits_{w=400}^{700} H_{red}(w)I(w)\rho_s(w)\,dw} \\[12pt] \dfrac{\int\limits_{w=400}^{700} H_{green}(w)I(w)PPG(w)\,dw}{\int\limits_{w=400}^{700} H_{green}(w)I(w)\rho_s(w)\,dw} \\[12pt] \dfrac{\int\limits_{w=400}^{700} H_{blue}(w)I(w)PPG(w)\,dw}{\int\limits_{w=400}^{700} H_{blue}(w)I(w)\rho_s(w)\,dw} \end{bmatrix}$$

which, using a white halogen illumination spectrum I(w), leads to a normalized PBV = [0.27, 0.80, 0.54]. When using a more noisy curve the result may be PBV = [0.29, 0.81, 0.50].

The blood volume pulse predicted by the used model corresponds reasonably well to an experimentally measured normalized blood volume pulse vector, PBV = [0.33, 0.78, 0.53] found after averaging measurements on a number of subjects under white illumination conditions. Given this result, it was concluded that the observed PPG-amplitude, particularly in the red, and to a smaller extent in the blue camera channel, can be largely explained by the crosstalk from wavelengths in the interval between 500 and 600 nm. The precise blood volume pulse vector depends on the color filters of the camera, the spectrum of the light and the skin-reflectance, as the model shows. In practice, the vector turns out to be remarkably stable though given a set of wavelength channels (the vector will be different in the infrared compared to RGB-based vector).

It has further been found that the relative reflectance of the skin, in the red, green and blue channel under white illumination does not depend much on the skin-type. This is likely because the absorption spectra of the blood-free skin is dominated by the melanin absorption. Although a higher melanin concentration can increase the absolute absorption considerably, the relative absorption in the different wavelengths remains the same. This implies an increase of melanin darkens the skin but hardly changes the normalized color of the skin. Consequently, also the normalized blood volume pulse PBV is quite stable under white illumination. In the infrared wavelengths, the influence of melanin is further reduced as its maximum absorption occurs for short wavelengths (UV-light) and decreases for longer wavelengths.

The stable character of PBV can be used to distinguish color variations caused by blood volume change from variations due to alternative causes, i.e. the stable PBV can be used as a "signature" of blood volume change to distinguish their color variations. The known relative pulsatilities of the color channels PBV can thus be used to discriminate between the pulse-signal and distortions. The resulting pulse signal S using known methods can be written as a linear combination (representing one of several possible ways of "mixing") of the individual DC-free normalized color channels:

$$S = W\, C_n$$

with $WW^T = 1$ and where each of the three rows of the 3 x N matrix $C_n$ contains N samples of the DC-free normalized red, green and blue channel signals $R_n$, $G_n$ and $B_n$, respectively, i.e.:

$$\vec{R}_n = \frac{1}{\mu(\vec{R})}\vec{R} - 1, \quad \vec{G}_n = \frac{1}{\mu(\vec{G})}\vec{G} - 1, \quad \vec{B}_n = \frac{1}{\mu(\vec{B})}\vec{B} - 1.$$

Here the operator $\mu$ corresponds to the mean. Key difference between the different methods is in the calculation of the weighting vector W. In one method, the noise and the PPG signal may be separated into two independent signals built as a linear combination of two color channels. One combination approximated a clean PPG signal, the other contained noise due to motion. As an optimization criterion the energy in the pulse signal may be minimized. In another method a linear combination of the three color channels may be used to obtain the pulse signal.

The PBV method generally obtains the mixing coefficients using the blood volume pulse vector as basically described in US 2013/271591 A1 and the above cited paper of de Haan and van Leest. The best results are obtained if the band-passed filtered versions of $R_n$, $G_n$ and $B_n$ are used. According to this method the known direction of PBV is used to discriminate between the pulse signal and distortions. This not only removes the assumption (of earlier methods) that the pulse is the only periodic component in the video, but also eliminates assumptions on the orientation of the distortion signals. To this end, it is assumed as before that the pulse signal is built as a linear combination of normalized color signals. Since it is known that the relative amplitude of the pulse signal in the red, green and blue channel is given by PBV, the weights, $W_{PBV}$, are searched that give a pulse signal S, for which the correlation with the color channels $R_n$, $G_n$, and $B_n$ equals $P_{bv}$

$$\vec{S}C_n^T = k\vec{P}_{bv} \Leftrightarrow \vec{W}_{PBV} C_n C_n^T = k\vec{P}_{bv}, \tag{1}$$

and consequently the weights determining the mixing are determined by

$$\vec{W}_{PBV} = k\vec{P}_{bv} Q^{-1} \text{ with } Q = C_n C_n^T, \tag{2}$$

and the scalar k is determined such that $W_{PBV}$ has unit length. It is concluded that the characteristic wavelength dependency of the PPG signal, as reflected in the normalized blood volume pulse, PBV, can be used to estimate the pulse signal from the time-sequential RGB pixel data averaged over the skin area. This algorithm is referred to as the PBV method.

In another embodiment the APBV method is used to extract an SpO2 value from two or more different combinations of three wavelength channels, e.g. from [λ1, λ2], from [λ1, λ3], and/or from [λ1, λ2, λ3]. In the following some basic considerations with respect to the APBV method shall be briefly explained.

Instead of extracting features from the PPG waveforms, APBV determines SpO2 indirectly based on the signal quality of the pulse signals extracted with SpO2 'signatures'. This procedure can mathematically be described as:

$$\overbrace{}^{\vec{W}_{PBV}}$$

$$SpO_2 = \underset{SpO_2 \in \mathbf{SpO_2}}{\operatorname{argmax}} \; SNR\left(k\vec{P}_{bv}(SpO_2)[\mathbf{C_n C_n^T}]^{-1} \mathbf{C_n}\right),$$

(3)

where $C_n$ contains the DC-normalized color variations and scalar k is chosen such that $\vec{W}_{PBV}$ has unit length. The SpO2 signatures compiled in $\vec{P}_{bv}$ can be derived from physiology and optics. Assuming identical cameras the PPG amplitudes of N cameras can be determined by:

$$\vec{P}_{bv} = \left\| \begin{bmatrix} (\frac{AC}{DC})^1 \\ (\frac{AC}{DC})^2 \\ \vdots \\ (\frac{AC}{DC})^N \end{bmatrix} \right\| = \left\| \begin{bmatrix} \dfrac{\int_\lambda I(\lambda)F^1(\lambda)C(\lambda)PPG(\lambda)d\lambda}{\int_\lambda I(\lambda)F^1(\lambda)C(\lambda)\rho_s(\lambda)d\lambda} \\ \dfrac{\int_\lambda I(\lambda)F^2(\lambda)C(\lambda)PPG(\lambda)d\lambda}{\int_\lambda I(\lambda)F^2(\lambda)C(\lambda)\rho_s(\lambda)d\lambda} \\ \vdots \\ \dfrac{\int_\lambda I(\lambda)F^N(\lambda)C(\lambda)PPG(\lambda)d\lambda}{\int_\lambda I(\lambda)F^N(\lambda)C(\lambda)\rho_s(\lambda)d\lambda} \end{bmatrix} \right\|.$$

(4)

Here the PPG amplitude spectrum, PPG($\lambda$) can be approximated by a linear mixture of the light absorption spectra from the two most common variants of the main chromophore in arterial blood, hemoglobin; oxygenated (HbO2) and reduced (Hb):

$$PPG(\lambda) \approx \epsilon_{Hb}(\lambda)c_{Hb} + \epsilon_{HbO_2}(\lambda)c_{HbO_2} = (1 - SaO_2)\epsilon_{Hb}(\lambda) + SaO_2\epsilon_{HbO_2}(\lambda)$$
$$= \epsilon_{Hb}(\lambda) + SaO_2[\epsilon_{HbO_2}(\lambda) - \epsilon_{Hb}(\lambda)],$$

(5)

where it is assumed that the optical path length differences are negligible for 600 < $\lambda$ < 1000nm and SaO2 $\in$ [0, 1]. It is recognized that the wavelength-dependent effect of scattering could render this assumption invalid. When using two wavelengths, the ratio-of-ratios parameter R and the ratio of APBV parameter $\vec{P}_{bv}$ coincide. The wavelength selection may be based on three criteria: 1) the desire to measure oxygen saturation in darkness ($\lambda$ > 700nm) for clinical applications, 2) have a reasonable SpO2 contrast, and 3) wavelengths within the spectral sensitivity of the camera. The idea to use three instead of the common two wavelengths used in pulse-oximetry was motivated by the improved robustness of the SpO2 measurement by a factor of two. This can be explained by how motion affects the PPG waveforms when measured with a camera. Since motion-induced intensity variations are equal for all wavelengths, suppression of these artifacts is possible for the APBV method if the pulse signature $\vec{P}_{bv}$ is not equal to this motion signature, which can be described as a vector with equal weights.

It shall be noted that even if the pulse quality is very good, it does not always mean that the estimated SpO2 value is sufficiently reliable and can be trusted. This may particularly happen when unexpected blood-species (e.g. COHb) are available causing the SpO2 calibration curve to shift, i.e. causing a different signature vector to lead to the optimal pulse quality when using the PBV method or APBV method for pulse extraction.

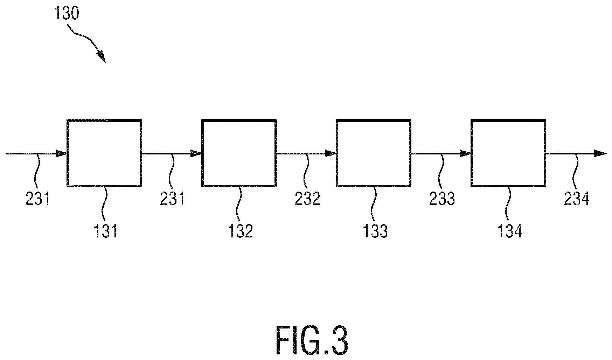

Fig. 3 shows a more detailed schematic illustration of an embodiment of the device 130 according to the present invention. The device 130 comprises an input interface 131 for obtaining (i.e. retrieving or receiving) at least three detection signals 231 derived from detected electromagnetic radiation transmitted through or reflected from a skin region of the subject 120. The data stream of detection data, i.e. the detection signals 231, is e.g. provided by the camera 112 and/or one or more PPG sensor(s) 114, wherein each detection signal comprises wavelength-dependent reflection or transmission information in a different wavelength channel.

A transformation unit 132 transforms the at least three detection signals 231 into at least three transformed detection signals 232 by temporally normalizing the at least three detection signals 231 or applying a non-linear transfer function on the at least three detection signals 231. For normalization time samples of the individual detection signals may be divided by a division value derived from said time samples to determine the at least three transformed detection signals. Alternatively, a logarithm function to determine the at least three transformed detection signals.

A projection unit 133 projects the at least three transformed detection signals 232 into at least two projected signals 233 in a subspace (substantially) orthogonal to a predetermined distortion direction in order to eliminated distortions induced by BCG motion and/or respiratory motions of the skin. Preferably, the at least three transformed detection signals 232 are projected into the at least two projected signals 233 by optimizing the selection of projection axes using as optimization criteria that the relative pulsatilities of the at least two projected signals are affected at all and that they are affected significantly differently by variations of the vital sign.

A vital sign determination unit 134 determines a vital sign 234 from said at least two projected signals 233 by indirectly determining the relative strength of a pulsatile component in said at least two projected signals 233. For this purpose, a blood-volume pulse vector based PBV or adaptive PBV method may be used.

The various units of the device 13 may be comprised in one or multiple digital or analog processors depending on how and where the invention is applied. The different units may completely or partly be implemented in software and carried out on a personal computer connected to one or more detectors. Some or all of the required functionality may also be implemented in hardware, e.g. in an application specific integrated circuit (ASIC) or in a field programmable gate array (FPGA).

That measurements with two and three wavelengths exhibit differences in behavior during patient movements already becomes evident when analyzing Fig. 1 in more detail. Since the relative pulsatility in the wavelengths shown depends on the oxygen saturation of the blood, it can be measured using two different wavelength-channels. Different wavelength channels may be sensitive for different wavelength intervals, or for the same wavelength interval, but with a different contribution from individual wavelengths. When using 760nm and 800nm as wavelengths, it can be seen from Fig. 1 that the relative pulsatilities become identical around a blood oxygen saturation of 78%. When using 760nm and 905nm as wavelengths, the relative pulsatilities become identical around a blood oxygen saturation of about 55% (the point lies outside of Fig. 1). When using three wavelengths, e.g. as proposed in the above cited paper of M. van Gastel, S. Stuijk and G. de Haan, "New principle for measuring arterial blood oxygenation, enabling motion-robust remote monitoring", Nature Scientific Reports, Nov. 2016 it can be seen from Fig. 1 that equal pulsatility cannot occur, which makes the system more robust for motion but at the same time leaves the outcome more unpredictable should excessive motion-induced distortions occur.

In a preferred embodiment of the system according to the present invention, a camera with three wavelength channels, e.g. 760nm, 800nm and 840nm is used. These wavelengths allow measurement in relative darkness, replacing 760nm by 660nm increases visibility of the illumination unit, but also improves the accuracy. Hence, the exact wavelengths are a compromise that may be selected depending on the application. Also, more wavelengths may be selected. From the three channels, corresponding (PPG) detection signals C1, C2, C3 are obtained, e.g. by segmenting out the skin pixels in each image, averaging them to a sample values, and concatenating said sample values to provide a detection signal for each wavelength. These detection signals are time-normalized, or their logarithm is taken, to obtain three transformed detection signals Ct1, Ct2, Ct3.

Next, in this embodiment, the mean is subtracted and two projection signals $P_1$, $P_2$ are computed, e.g. using: $P_1$ = [-1.0465 0.9459 0.1006]*[Ct1 Ct2 Ct3] and $P_2$ = [-0.8452 -1.1472 1.9924]* [Ct1 Ct2 Ct3]. This specific projection may found by rotating an initial projection on the POS (plane in which all projections with coefficients adding up to zero are located) and optimizing for the following two equally weighted criteria within the oxygen saturation range [60% - 100%]:

  a) Pulse amplitude assuming IID (independent and identically distributed) noise (since APBV is only robust to linear, correlated distortions), and
  b) SpO2 contrast (to be able to detect small variations in SpO2).

Other criteria can be used, and also equal weighting can be modified to give priority to one criterion over the other.

Finally, the SpO2 estimate results from applying the APBV-method to the two projected signals, i.e. a set of pre-computed signature vectors (PBV vectors computed for the projected space) is used to extract a PPG pulse signal for each PBV vector using the PBV method as e.g. described in the above cited paper of G. de Haan and A. van Leest, to compare the quality of the resulting PPG-pulse signals (SNR, signal amplitude, spectral flatness, spectral skewness, etc.), and to use the PBV vector of the best quality pulse signal to find the SpO2 value as described in M. van Gastel, S. Stuijk and G. de Haan, "New principle for measuring arterial blood oxygenation, enabling motion-robust remote monitoring", Nature Scientific Reports, Nov. 2016.

In the following, more details of an exemplary implementation of the proposed system and method will be described. In this implementation, a camera is used as detection unit to acquired video frames over time, the pixel signals of the video frames representing the detection signals. From the video frames, SpO2 values shall be estimated in this implementation from different anatomical locations, particularly with high pulsatile amplitudes on both the face and hand, mouth, forehead, cheeks, full face and palm. For the face, facial landmarks are detected from the full-frame image which are subsequently used to generate ROIs using polygonization. The pixels within the ROIs are averaged for the three color channels to overcome sensor and quantization noise. For the hand, the palm may be manually selected at initialization.

A cardiac pulse signal can be expressed as a linear combination of DC-normalized color channels contaminated with noise:

$$\tilde{\mathbf{S}} = \vec{W}\mathbf{C_n} + \eta,$$

(6)

where $\vec{W}$ denotes the weights and $C_n$ is a matrix containing the DC-normalized color signals. To find the weights for $\vec{W}$, various methods have been proposed, which can roughly classified into i) BSS-based (blind signal source based) methods using heuristics to select the pulse signal from the other signals, ii) methods which exploit the known characteristics of the typical distortions, and iii) methods using prior knowledge about the relative pulse amplitudes. Since the prior of the later approach is SpO2-dependent the method can be extended to enable robust SpO2 measurements:

$$SpO_2 = \underset{SpO_2 \in \mathbf{SpO_2}}{\mathrm{argmax}} \; SNR\left( \overbrace{k\vec{P_{bv}}(SpO_2)[\mathbf{C_n}\mathbf{C_n^T}]^{-1}\mathbf{C_n}}^{\vec{W}_{PBV}} \right),$$

(7)

where $\vec{P}_{bvl}$ ($SpO_2$) denotes the pulse vector and $k$ is a scalar to scale the weights to unit length. Instead of extracting features from the individual PPG waveforms, a single pulse signal is created as a linear combination of the PPG waveforms using the SpO2 -dependent pulse 'signature' prior. The benefit of this approach is the ability to suppress distortions enabling robust pulse rate detection whilst the optimum corresponding to the SpO2 value remains stable. Essentially, by searching for the pulse vector that provides the best pulse signal in terms of SNR, the oxygen saturation can be estimated. A consequence of the SNR-based selection criterion is that cardiac-similar frequencies, e.g. ballistocardiography (BCG), influence the measurement. Under uniform, homogeneous illumination it is known that such motion causes intensity variations equal in all color channels, which can be expressed by the mixing vector 1. A projection on a plane orthogonal to this distortion may thus be applied on the DC-normalized signals to eliminate motion artifacts before oxygenation levels are estimated. This projection should therefore satisfy:

$$\mathbf{P} \cdot \vec{1} = 0.$$

(8)

This projection should consequently also be applied to the SpO2 calibration model, $\vec{P}_{bvl}$ ($SpO_2$), used for pulse extraction:

$$SpO_2 = \underset{SpO_2 \in \mathbf{SpO_2}}{\mathrm{argmax}} \; SNR\left( \overbrace{k\mathbf{P}\vec{P_{bv}}(SpO_2)[\mathbf{Cp_n}\mathbf{Cp_n^T}]^{-1}\mathbf{Cp_n}}^{\vec{W}_{PBV}} \right),$$

(9)

where $\mathbf{Cp_n}$ = $\mathbf{PC_n}$. Consequences of the projection are that the pulse amplitudes are lower compared to the amplitudes before projection and that noise is boosted. Depending on the projection, the two projected signals could also be in anti-phase for certain oxygen saturations. This may be problematic for the conventional ratio-of-ratios method but it has a much less detrimental effect on the performance of APBV. Since there is the freedom to use any projection on the plane orthogonal to **1,** the projection may be determined based on two criteria: a) pulse amplitude and b) SpO2 contrast, indicated with $F^p$ and $F^c$, respectively:

$$F^p(\Theta) = \overline{\left( \sum_{SpO_2 \in \mathbf{SpO_2}} |R(\theta)\mathbf{P}\vec{P}_{bv}(SpO_2)| \right)}$$

$$F^c(\Theta) = \overline{\left( \frac{\partial}{\partial S} \frac{P_{bv}^{p,1}}{P_{bv}^{p,2}} \right)}, \text{with } \begin{bmatrix} P_{bv}^{p,1} \\ P_{bv}^{p,2} \end{bmatrix} = R(\theta)\vec{P}_{bv}(SpO_2) \quad \forall\, SpO_2 \in \mathbf{SpO_2},$$

$$(10)$$

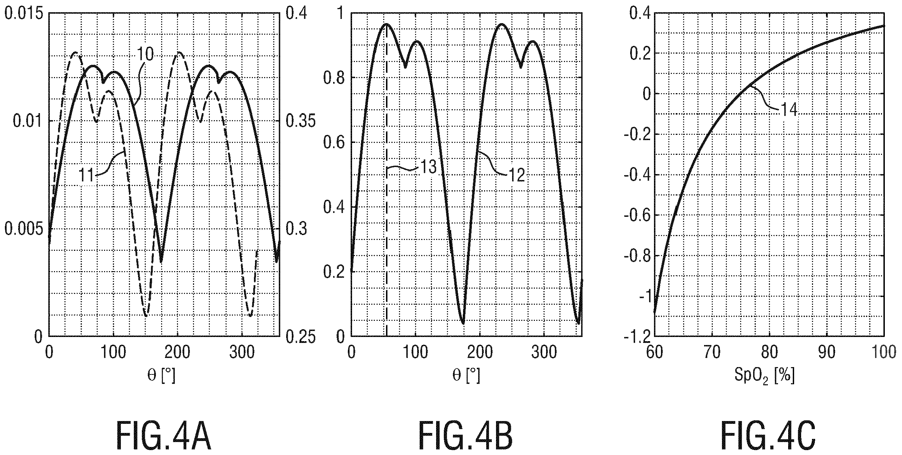

where $^{-}$ is the sample mean operator and $\partial/\partial S$ the derivative with respect to oxygenation levels. Since APBV can only cope with linear relations such as pulse and motion, and not with uncorrelated noise, e.g. sensor noise, it is preferred to have signals with reasonable pulse amplitude after projection. Furthermore, it is preferred to have a reasonable SpO2 contrast, which is specified as the change in absorption as a function of the oxygen saturation levels. A large contrast allows to detect small changes in oxygen saturation. In order to find the optimal projection, an initial projection is rotated on the plane orthogonal to **1** and the values of the applied criteria are evaluated, as visualized in Fig. 4.

Fig. 4A shows values of the two optimization criteria average contrast (curve 10) and average amplitude (curve 11) for rotations on the plane orthogonal to the intensity variations. Fig. 4B shows the value 12 of the combined, equally weighted optimization features where the dashed line 13 indicates the optimum. Fig. 4C shows the calibration curve 14 for the optimal projection.

As already mentioned above, when giving equal weights to both optimization criteria and evaluating oxygen saturation levels in the expected range [60 - 100]%, the optimal projection is found to be

$$\mathbf{P} = \begin{bmatrix} -1.0465 & 0.9459 & 0.1006 \\ -0.8452 & -1.1472 & 1.9924 \end{bmatrix}$$

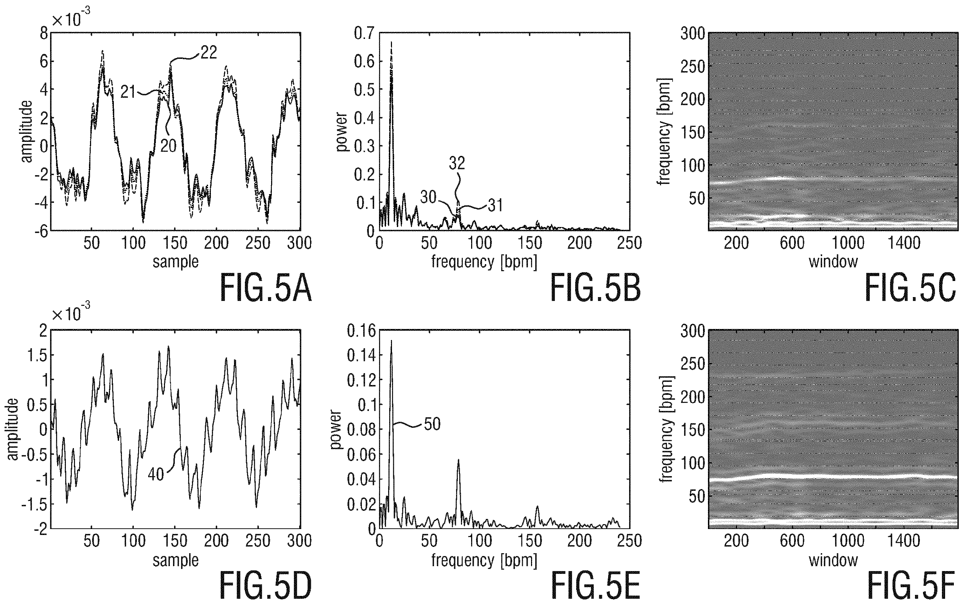

An illustration of this projection applied on data obtained on the forehead is visualized in Fig. 5. Fig. 5A shows DC-normalized signals 20 (760nm), 21 (800nm), 22 (890nm) from the three channels. Fig. 5B shows the corresponding spectra 30 (760nm), 31 (800nm), 32 (890nm) with peaks at both the breathing and pulse rate. Fig. 5C shows a spectrogram of the 890nm channel from the full recording. Fig. 5D shows a signal 40 after projection to eliminate motion artifacts. Fig. 5E shows a corresponding spectrum 50 after amplitude tuning. Fig. 5F shows a spectrogram from the full recording.

The projection is applied to two differently band-pass filtered versions of the DC-normalized signals; a) filtered for respiration, and b) filtered for pulse. Respiratory frequencies lower than 10 breaths/min are eliminated since these interfere with Traube-Hering-Mayer (THM) waves, whereas higher frequencies interfere with pulse. Estimates of arterial saturation levels are finally obtained using the above described APBV method (see also equation (9)). In an implementation time-windows of 20 seconds, a step-size of 1 second and a lOth-order Butterworth filter for band-pass filtering may be applied.

The above described methods can be applied on detection signals that have been acquired using contact sensors and/or contactless sensors. By way of example, the present invention can be applied in the field of healthcare, e.g. unobtrusive remote patient monitoring, general surveillances, security monitoring and so-called lifestyle environments, such as fitness equipment, fitness/health-watches/bands (typically wrist-worn), or the like. Applications may include monitoring of oxygen saturation (pulse oximetry), pulse rate, blood pressure, cardiac output, changes of blood perfusion, assessment of autonomic functions, respiration and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward, and offer better solutions for motion robustness.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining at least one vital sign of a subject, said device comprising:

- an input interface (131) configured to obtain at least three detection signals derived from detected electromagnetic radiation transmitted through or reflected from a skin region of a subject, wherein each detection signal comprises wavelength-dependent reflection or transmission information in a different wavelength channel,
- a transformation unit (132) configured to transform the at least three detection signals into at least three transformed detection signals by temporally normalizing the at least three detection signals or applying a non-linear transfer function on the at least three detection signals,
- a projection unit (133) configured to project the at least three transformed detection signals into at least two projected signals in a subspace orthogonal to a predetermined distortion direction, and
- a vital sign determination unit (134) configured to determine a vital sign from said at least two projected signals by indirectly determining the relative strength of a pulsatile component in said at least two projected signals.

2. Device as claimed in claim 1,
wherein said projection unit (133) is configured to project the at least three transformed detection signals into the at least two projected signals to optimize the sensitivity of the vital sign and the strength of the pulsatile component.

3. Device as claimed in claim 2,
wherein said projection unit (133) is configured to project the at least three transformed detection signals into the at least two projected signals by optimizing the selection of projection axes using as optimization criteria that the relative pulsatilities of the at least two projected signals are affected at all and that the relative pulsatilities are affected significantly differently by variations of the vital sign.

4. Device as claimed in claim 1,
wherein said transformation unit (132) is configured to divide time samples of the individual detection signals by a division value derived from said time samples to determine the at least three transformed detection signals.

5. Device as claimed in claim 1,
wherein said transformation unit (132) is configured to transform time samples of the detection signals using a logarithm function to determine the at least three transformed detection signals.

6. Device as claimed in claim 1,
wherein the wavelength channels are in a wavelength interval between 400nm and 1000nm, in particular between 630nm and 940nm.

7. Device as claimed in claim 1,
wherein said input interface (131) is configured to obtain three detection signals in wavelength channels centered on i) 660nm or 760nm, ii) 800nm, and iii) 840nm or 900nm.

8. Device as claimed in claim 1,
wherein said projection unit (133) is configured to project the at least three transformed detection signals into at least two projected signals by a weighted summation of the transformed detection signals, wherein the sum of the weights equals zero.

9. Device as claimed in claim 1,
wherein said projection unit (133) is configured to project three transformed detection signals Ct1, Ct2, Ct3 into two projected signals $P_1$, $P_2$ as:

$$P_1 = [-1.0465\ 0.9459\ 0.1006] * [Ct1\ Ct2\ Ct3];$$

$$P_2 = [-0.8452\ -1.1472\ 1.9924] * [Ct1\ Ct2\ Ct3].$$

**10.** Device as claimed in claim 1,
wherein said vital sign determination unit (134) is configured to indirectly determine the relative strength of a pulsatile component in said at least two projected signals by use of a blood-volume pulse vector based PBV or adaptive PBV method.

**11.** Device as claimed in claim 1,
wherein said vital sign determination unit (134) is configured to determine as vital sign an indicator or concentration of blood components or species, in particular SpO2, SvO2, HBO2, HBCO, METHB, Bilirubin, and wherein the pulsatile component reflects the cardiac cycle or the respiratory cycle.

**12.** System for determining at least one vital sign of a subject, said system comprising:

- a detector (110; 112, 114) for detecting electromagnetic radiation transmitted through or reflected from a skin region of a subject and for deriving at least three detection signals (210) from the detected electromagnetic, wherein each detection signal comprises wavelength-dependent reflection or transmission information in a different wavelength channel, and
- a device (130) as claimed in claim 1 for determining at least one vital sign of the subject from the at least three detection signals.

**13.** Method for determining at least one vital sign of a subject, said method comprising:

- obtaining at least three detection signals derived from detected electromagnetic radiation transmitted through or reflected from a skin region of a subject, wherein each detection signal comprises wavelength-dependent reflection or transmission information in a different wavelength channel,
- transforming the at least three detection signals into at least three transformed detection signals by temporally normalizing the at least three detection signals or applying a non-linear transfer function on the at least three detection signals,
- projecting the at least three transformed detection signals into at least two projected signals in a subspace orthogonal to a predetermined distortion direction, and
- determining a vital sign from said at least two projected signals by indirectly determining the relative strength of a pulsatile component in said at least two projected signals.

**14.** Computer program comprising program code means for causing a computer to carry out the steps of the method as claimed in claim 13 when said computer program is carried out on the computer.

FIG.1

FIG.2

FIG.3

FIG.4A   FIG.4B   FIG.4C

FIG.5A   FIG.5B   FIG.5C   FIG.5D   FIG.5E   FIG.5F

Europäisches
Patentamt

European
Patent Office

Office européen
des brevets

# EUROPEAN SEARCH REPORT

Application Number

EP 18 15 3213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG WENJIN ET AL: "Algorithmic Principles of Remote PPG", IEEE TRANSACTIONS ON BIOMEDICAL ENGINEERING, IEEE SERVICE CENTER, PISCATAWAY, NJ, USA, vol. 64, no. 7, 1 July 2017 (2017-07-01), pages 1479-1491, XP011653906, ISSN: 0018-9294, DOI: 10.1109/TBME.2016.2609282 [retrieved on 2017-06-15] * page 1479, right-hand column, paragraph 2 * * page 1479, right-hand column, paragraph 4 - page 1481, left-hand column, paragraph 1 * * page 1481, right-hand column, paragraph 2 - page 1482, left-hand column, paragraph 2 * * page 1483, left-hand column, last paragraph - page 1486, left-hand column, paragraph 1 * * page 1486, right-hand column, last paragraph * * page 1488, left-hand column, paragraph 3 * * figures 1-5 * | 1-8, 10-14 | INV. A61B5/1455 A61B5/00 A61B5/024 |
| X | US 2012/195486 A1 (KIRENKO IHOR OLEHOVYCH [NL] ET AL) 2 August 2012 (2012-08-02) * paragraph [0026] * * paragraph [0028] * * paragraph [0033] * * paragraph [0036] - paragraph [0037] * * paragraph [0071] - paragraph [0078] * * figures 1-6 * * paragraph [0086] - paragraph [0087] * * paragraph [0111] - paragraph [0112] * | 1-8, 10-14 | TECHNICAL FIELDS SEARCHED (IPC) A61B |

-/--

The present search report has been drawn up for all claims

| Place of search | Date of completion of the search | Examiner |
|---|---|---|
| Berlin | 13 July 2018 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 15 3213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE HAAN GERARD ET AL: "Robust Pulse Rate From Chrominance-Based rPPG", IEEE TRANSACTIONS ON BIOMEDICAL ENGINEERING, IEEE SERVICE CENTER, PISCATAWAY, NJ, USA, vol. 60, no. 10, 1 October 2013 (2013-10-01), pages 2878-2886, XP011526965, ISSN: 0018-9294, DOI: 10.1109/TBME.2013.2266196 [retrieved on 2013-09-16] * page 2878, right-hand column, last paragraph - page 2880, left-hand column, paragraph 2 * * page 2881, left-hand column, paragraph 1 * | 1-14 | |
| A,D | G. DE HAAN; A. VAN LEEST: "Improved motion robustness of remote-PPG by using the blood volume pulse signature", PHYSIOL. MEAS., vol. 35, 2014, page 1913, XP20269523, * page 1915, paragraph 2 - page 1920, paragraph 1 * | 1-14 | TECHNICAL FIELDS SEARCHED (IPC) |

The present search report has been drawn up for all claims

| Place of search | Date of completion of the search | Examiner |
|---|---|---|
| Berlin | 13 July 2018 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 3213

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2018

| Patent document cited in search report | Publication date | Patent family member(s) | Publication date |
|---|---|---|---|
| US 2012195486 A1 | 02-08-2012 | CN 102576458 A | 11-07-2012 |
| | | EP 2486539 A1 | 15-08-2012 |
| | | EP 3142067 A2 | 15-03-2017 |
| | | JP 5856960 B2 | 10-02-2016 |
| | | JP 2013506523 A | 28-02-2013 |
| | | US 2012195486 A1 | 02-08-2012 |
| | | US 2014153800 A1 | 05-06-2014 |
| | | US 2015104088 A1 | 16-04-2015 |
| | | WO 2011042839 A1 | 14-04-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150366492 A1
- WO 2015197383 A1
- US 2013271591 A1

**Non-patent literature cited in the description**

- **G. DE HAAN ; A. VAN LEEST.** Improved motion robustness of remote-PPG by using the blood volume pulse signature. *Physiol. Meas.,* 2014, vol. 35, 1913
- **M. VAN GASTEL ; S. STUIJK ; G. DE HAAN.** New principle for measuring arterial blood oxygenation, enabling motion-robust remote monitoring. *Nature Scientific Reports,* November 2016
- **F M. VAN GASTEL ; S. STUIJK ; G. DE HAAN.** New principle for measuring arterial blood oxygenation, enabling motion-robust remote monitoring. *Nature Scientific Reports,* November 2016